# EUROPÄISCHE PATENTSCHRIFT

(11) **EP 1 202 698 B1**
(45) Veröffentlichungstag und Bekanntmachung des Hinweises auf die Patenterteilung: **22.10.2003**
(21) Anmeldenummer: 00954385.1
(22) Anmeldetag: 28.07.2000
(51) Int. Cl.: A61H 7/00

(54) **THERAPIEBANDAGE**
THERAPEUTIC BANDAGE
BANDAGE THERAPEUTIQUE

(30) Priorität: 09.08.1999 DE 19937535
(43) Veröffentlichungstag der Anmeldung: 08.05.2002
(73) Patentinhaber: Hahn, Matthias P.-G., 67731 Ottersbach (DE)
(72) Erfinder: Hahn, Matthias P.-G., 67731 Ottersbach (DE)
(74) Vertreter: Becker, Bernd, Dipl.-Ing.
(86) Internationale Anmeldenummer: DE0002494
(87) Internationale Veröffentlichungsnummer: WO01010370

(56) Entgegenhaltungen:
- WO-A-97/49306
- DE-A- 4 219 698
- DE-C- 82 289
- FR-A- 1 367 724
- US-A- 5 381 558
- US-A- 5 607 749
- US-A- 5 769 803

## Beschreibung

Die Erfindung bezieht sich auf eine Therapiebandage mit mindestens einer Therapiefläche für ein zu behandelndes Körperteil, auf der in Richtung des zu behandelnden Körperteiles weisende Massageansätze angeordnet sind, wobei die Therapiebandage mittels eines Verschlusses an dem Körperteil lösbar festgelegt ist.

Die DE-A-196 11 888 offenbart eine doppelwandig ausgeführte Stützbandage, die den zu stützenden Körper im Benutzungszustand eng anliegend umgreift, wobei die Bandage unter Vorspannung setzbar ist, indem sich an einem größeren Bandageabschnitt,. der im Tragezustand gegenüber dem Rücken des Trägers liegt, Spannbänder anschließen, die das Stützteil unter Vorspannung setzen und aus einem die Vorspannung erzeugenden Material, wie Gummi, Kunststoff oder dergleichen bestehen.

Darüber hinaus zeigt die DE-C-197 25 648 eine flexible orthopädische Bandage, die im offenen Zustand um ein Körperteil legbar und dann mittels eines Schnellverschlusses verschließbar ist. Im Bereich des Schnellverschlusses weist die Bandage ein Mittel zur vorübergehenden Versteifung dieses Bereiches während des Öffnens und/oder Schließens der Bandage auf.

Die vorgenannten Bandagen dienen lediglich zum Abstützen eines geschwächten Körperteiles. Eine weitergehende unterstützende Behandlung dieses Körperteiles ist nicht möglich. Hierzu werden üblicherweise Massagegenstände, wie beispielsweise Bürsten, Bälle, Handschuhe und dergleichen, verwendet, mit denen das zu behandelnde Körperteil bei Bedarf massiert wird. Die Verwendung dieser Massagegenstände erweist sich allerdings für den Benutzer oftmals als sehr anstrengend und ist daher auf eine relativ kurze Zeitspanne begrenzt. Schwer erreichbare Körperzonen, wie z.B. bestimmte Rückenpartien, lassen sich nur mit Hilfe einer weiteren Person behandeln.

Im Weiteren ist aus der DE 42 19 698 A1 eine Therapiebandage zur Druckbeaufschlagung eines Körperteils mit zumindest einer an das betreffende Körperteil anlegbaren Druckauflage und mindestens einer Halteeinrichtung zum fixierenden Halten der Druckauflage unter einem statischen Druck bekannt. Die Druckauflage weist einzelne Kugeln als Massageansätze auf, die zur Übertragung von von Kräften in eine Silikonmasse eingebettet sind. Die Massageansätze sind zur Erzeugung von im Wesentlichen diskret auf den betreffenden Körperteil wirkenden Reaktionskräften durch Muskelkraft beaufschlagbar. Die über die Silikonmasse hervorstehenden Abschnitte der Massageansätze weisen die Form balliger Noppen auf.

Darüber hinaus offenbart die US-A-7 769 803 eine Therapiebandage, die mittels eines Verschlußes lösbar an einem zu behandelnden Körperteil festzulegen ist. Zur Stimulierung des Körperteils ist die Therapiebandage mit balligen Massageansätzen versehen, die an dem zu therapierenden Körperteil zur Anlage kommen. Weiterhin ist aus der US-A-5 381 558 und der WO 97/49306 ein Bekleidungsstück bekannt, das Massageansätze aufweist, die auf einem zu massierenden Körperteil zur Anlage kommen.

Des Weiteren offenbart die FR-A-1 367 724 eine Massagebandage mit einem Hohlraum für ein Therapeutikum. Aus der DE 82 289 C ist eine Therapiebandage bekannt, die im Bereich von Massageansätzen mit Reizstrom beaufschlagbar ist. Ferner offenbart die US-A-5 607 749 eine als Flächenbandage ausgebildete Therapiebandage mit Massageansätzen.

Schließlich sind sogenannte Gesundheitspflaster bekannt, die auf das zu behandelnde Körperteil aufgebracht werden und ein Therapeutikum abgeben. Nach einer gewissen Zeitspanne ist das Therapeutikum aufgebraucht und das Gesundheitspflaster muß im Bedarfsfall durch ein neues ersetzt werden. Oftmals führt die Benutzung aufgrund einer Unverträglichkeit der Haut mit einem auf das Gesundheitspflaster aufgebrachten Klebstoff zu einer allergischen Reaktion des zu behandelnden Körperteiles.

Es ist Aufgabe der Erfindung, eine Therapiebandage der eingangs genannten Art zu schaffen, die eine gezielte langandauernde Massagebehandlung eines Körperteiles ermöglicht.

Erfindungsgemäß wird die Aufgabe dadurch gelöst, daß die Massageansätze als Bürsten ausgebildet sind.

Die als Bürsten ausgebildeten Massageansätze entfalten ihre therapeutische Wirkung während der gesamten Zeitspanne, in der die Therapiebandage an dem zu behandelnden Körperteil festgelegt ist. Die Massage erfolgt für den Träger der Therapiebandage ermüdungsfrei und ohne die Hilfe weiterer Personen, und zwar lediglich durch die Bewegungen, die der Träger mitsamt der Therapiebandage ausführt, wobei die Verwendung von Bürsten ist eine relativ großflächige Auflage auf dem zu behandelnden Körperteil sicherstellt. Die Wirkung der Bürsten ist durch eine gezielte Auswahl der Härte der Borsten einzustellen. Nach dem ordnungsgemäßen Festlegen der Therapiebandage werden lediglich die bewegten Körperteile massiert, weshalb eine Überreizung durch die Massageansätze nahezu ausgeschlossen werden kann. Zur Unterstützung der Wirkung der Bürsten kann zusätzlich ein Therapeutikum von Hand entweder direkt auf die Haut oder auf die Bürsten aufgetragen werden, das beim Tragen der Therapiebandage einmassiert wird.

Alternativ wird die Aufgabe bei einer Therapiebandage mit mindestens einer Therapiefläche für ein zu behandelndes Körperteil, auf der in Richtung des zu behandelnden Körperteiles weisende, als ballige Noppen ausgebildete Massageansätze angeordnet sind, wobei die Therapiebandage mittels eines Verschlusses an dem Körperteil lösbar festgelegt ist. erfindungsgemäß dadurch gelöst, dass auf der Kuppe des Noppens eine Akupunkturspitze angeordnet ist.

Durch die Akupunkturspitze ist eine intensive Reizung bestimmter Stellen des zu behandelnden Körperteiles gewährleistet.

Bevorzugt ist jeder Noppen mindestens im Bereich seiner Kuppe steif ausgeführt. Aufgrund der steifen Ausbildung der Kuppe des Noppens läßt sich eine kräftige punktuelle Massage mit Tiefenwirkung erzielen.

Um ein schmerzhaftes Anpressen bzw. Gleiten auf der Haut zu verhindern, ist zweckmäßigerweise zwischen der Kuppe des Noppens und der Therapiefläche ein elastischer Übergangsbereich vorgesehen. Der elastische Übergangsbereich sorgt für einen stets angenehmen Anpreßdruck der an der Kuppe des Noppens angeordneten Akupunkturspitze an das zu behandelnde Körperteil.

Bevorzugt ist der Therapiefläche unter Ausbildung eines mit einem Zu-/Ablauf für ein Therapeutikum versehenen Hohlraumes eine Außenwand zugeordnet. Das durch den Hohlraum geleitete Therapeutikum, das beispielsweise warmes Wasser ist, verstärkt die Wirkung der Therapiebandage. Neben der Reizung durch die Massageansätze findet eine Entspannung des zu behandelnden Körperteiles aufgrund der durch die Therapiefläche abgestrahlten Wärme statt.

Damit ein als Salbe, Massageöl oder dergleichen verwendetes Therapeutikum aus dem Hohlraum auf die Haut des zu behandelnden Körperteiles gelangen kann, ist vorzugsweise jedem Noppen bzw. jeder Bürste eine mit dem Hohlraum verbundene Austrittsöffnung für das Therapeutikum zugeordnet. Das in dem Hohlraum vorhandene Therapeutikum wird somit kontinuierlich aus dem Noppen bzw. der Bürste ausgetragen und in die Haut einmassiert.

Zweckmäßigerweise ist jede Noppe bzw. Bürste in der Wandung der Therapiefläche gehaltert. Vorteilhafterweise ist jede Noppe bzw. Bürste mit einem Halter versehen, der ein an der Unterseite der Noppe bzw. Bürste angeformtes Kugelsegment, einen koaxial davon abgehenden, einen Dichtkegel tragenden Halteransatz sowie einen an den Dichtkegel anschließenden Druckkonus umfaßt, wobei in einem unbeaufschlagten Zustand der Noppe bzw. Bürste der Dichtkegel aufgrund einer auf den Druckkonus wirkenden Federmatte an einem in Richtung der Außenwand weisenden Dichtsitz der Therapiefläche anliegt, der Halteransatz mit Spiel von einer Ansatzbohrung aufgenommen ist und das Kugelsegment beabstandet zu einer Noppenschale in der Therapiefläche einliegt. Hierdurch ist eine Abdichtung der Therapiefläche gegenüber dem Hohlraum im unbeaufschlagten Zustand der Noppe bzw. Bürste mit einer geringen Anzahl von Bauteilen sichergestellt. Gleichzeitig wird im beaufschlagten Zustand der Noppe bzw. Bürste ein Durchlaß für das Therapeutikum aus dem Hohlraum über die Ansatzbohrung zu der Therapiefläche freigegeben und der Anpreßdruck der Noppe bzw. Bürste wird im wesentlichen durch die Federmatte bestimmt.

Nach einer vorteilhaften Weiterbildung des Erfindungsgedankens sind die Noppen Bestandteil einer elastischen, an der Therapiefläche befestigten Noppenfolie, wobei jedem Noppen ein in den Hohlraum mündender Kanal zugeordnet ist. Somit ist eine kostengünstige Herstellung der Therapiebandage sichergestellt, da die Noppenfolie mitsamt den Noppen ein in einem Fertigungsprozeß hergestelltes Einzelteil ist, das an der Therapiebandage befestigt wird. Durch den in den Hohlraum mündenden Kanal, wird die Festigkeit der Noppen in Abhängigkeit von dem im Hohlraüm herrschenden, regulierbaren Druck bestimmt.

Zweckmäßigerweise ist in den Kanal ein Ventil eingesetzt. Das Ventil verhindert eine selbsttätige Druckänderung innerhalb des zugeordneten Noppens. Vorteilhafterweise ist das Ventil federbelastet oder mit einer Rasteinrichtung ausgeführt. Das federbelastete Ventil ermöglicht eine druckabhängige Füllung des zugeordnete Noppens. Mittels der Rasteinrichtung ist dahingegen ein dauerhaftes Öffnen des Ventils gewährleistet.

Nach einer weiteren vorteilhaften Ausgestaltung der Erfindung sind im Bereich der Noppen über Zuleitungen mit Reizstrom beaufschlagbare Elektroden vorgesehen. Hierdurch ist eine Kombination verschiedener therapeutischer Maßnahmen, durch die oftmals ein schneller Heilungserfolg erzielt wird, möglich. Beispielsweise läßt sich ein Therapeutikum mittels der Noppen in die Haut einmassieren und gleichzeitig eine Behandlung mit Reizstrom vornehmen.

Durch die zweckmäßige Ausbildung als Flächenbandage, läßt sich mit der Therapiebandage ein relativ großes zu behandelndes Körperteil, wie beispielsweise die Bauch- oder Rückenpartie, überdecken. Eine für den Träger der Therapiebandage besonders bequeme sowie großflächige wirkende Therapiebandage wird nach einer Weiterbildung der Erfindung durch die Ausbildung als als Weste, Hose oder Manschette bereitgestellt.

Da das Festlegen der Therapiebandage mit möglichst geringem Aufwand erfolgen soll, ist vorteilhafterweise der Verschluß als mindestens mit einem Knopf zusammenwirkende Knopflochleiste oder als Klettverschluß ausgebildet.

Es versteht sich, daß die vorstehend genannten und nachstehend noch zu erläuternden Merkmale nicht nur in der jeweils angegebenen Kombination, sondern auch in anderen Kombinationen verwendbar sind, ohne den Rahmen der vorliegenden Erfindung zu verlassen.

Die Erfindung wird im folgenden anhand meherer Ausführungsbeispiele unter Bezugnahme auf die zugehörigen Zeichnungen näher erläutert. Es zeigen:
- Fig.1: eine Darstellung einer erfindungsgemäßen Therapiebandage in einer ersten Ausführungsform,
- Fig.2: eine Darstellung nach Fig. 1 in einer ersten alternativen Ausführungsform,
- Fig.3: eine Darstellung nach Fig. 1 in einer zweiten alternativen Ausführungsform,
- Fig.4: eine Darstellung nach Fig. 1 in einer dritten alternativen Ausführungsform,
- Fig.5: eine schematische Darstellung der Ausführungsform nach Fig. 4,
- Fig. 6: eine Darstellung eines Schnittes gemäß der Linie VI-VI nach Fig. 2,
- Fig.7: eine Darstellung eines Schnittes gemäß der Linie VI-VI nach Fig. 2 in einer alternativen Ausgestaltung,
- Fig.8: eine vergrößerte Darstellung einer Einzelheit VIII nach Fig. 6 in einer alternativen Ausgestaltung,
- Fig.9: eine vergrößerte Darstellung einer Einzelheit IX nach Fig. 7. in einer ersten alternativen Ausgestaltung,
- Fig.10: eine vergrößerte Darstellung einer Einzelheit IX nach Fig. 7 in einer zweiten alternativen Ausgestaltung,
- Fig.11: eine vergrößerte Darstellung einer Einzelheit IX nach Fig. 7 in einer dritten alternativen Ausgestaltung und
- Fig.12: eine vergrößerte Darstellung einer Einzelheit XII nach Fig. 11.

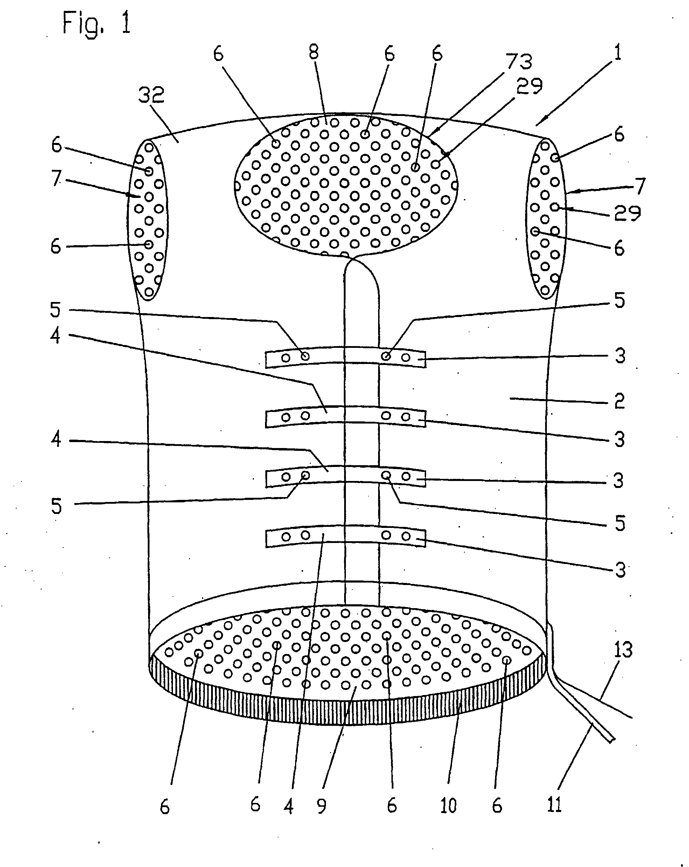

Die Fig. 1 zeigt eine als Weste 2 ausgebildete Therapiebandage 1, die der Körperform eines Trägers angepaßt ist und über vier Verschlüsse 3 im vorderen Bereich zusammengehalten wird. Die Verschlüsse 3 sind jeweils als mit einem an der Weste 2 befestigten Knopf 5 zusammenwirkende Knopflochleiste 4 ausgeführt, weshalb die Weste 2 an unterschiedliche Körpergrößen anpassbar ist. Im weiteren ist die Weste 2 mit zwei Armdurchführungen 7 versehen und weist im geschlossenen Zustand einen Halsdurchtritt 8 sowie eine Bundöffnung 9 auf, wobei die Bundöffnung 9 von einem Bund 10 gebildet wird, von dem ein Zu-/ Ablauf 11 für ein Therapeutikum sowie eine Reizstrom führende Zuleitung 13 abgehen. Der gesamte Innenbereich der Weste 2 bildet eine Therapiefläche 73, auf der in Richtung des zu behandelnden Körperteiles 76 weisende, als Noppen 6 ausgebildete Massageansätze 29 angeordnet sind.

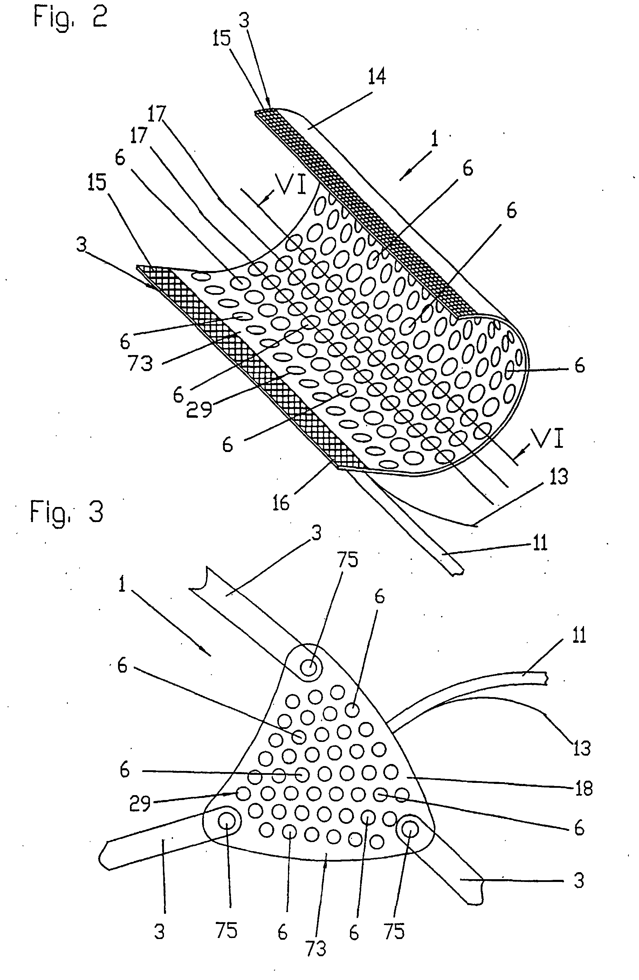

Die in Fig. 2 dargestellte Therapiebandage 1 ist eine Manschette 18, die über einen als Klettverschluß 15 ausgebildeten Verschluß 3 am zu behandelnden Körperteil 76 fixiert wird. Im Bereich eines Klettenbandes 16 des Klettverschlußes 15 ist der von einer gemeinsamen Umhüllung umgebene Zu-/Ablauf 11 sowie die Zuleitungen 13 eingelassen. Auf der nach innen gerichteten Therapiefläche 73 der Manschette 18 sind wiederum in versetzt zueinander angeordneten Reihen 17 ballige Noppen 6 befestigt.

Fig. 3 zeigt eine als Flächenbandage 18 gestaltete Therapiebandage 1, die eine dreieckige Form aufweist, an deren Spitzen über Gelenke 75 Verschlüsse 3 angebracht sind, mittels denen die Flächenbandage 18 am Körper fixiert wird. Aufgrund der Gelenke 75 lassen sich die Verschlüsse 3 in einer an den Körper angepaßten Richtung verschwenken. Auf der in Richtung des zu behandelnden Körperteiles 76 weisenden, nach innen gerichteten Therapiefläche 73 sind ballige Noppen 6 angeordnet. Einer rückseitigen Außenwand 32 der Therapiebandage 1 ist der Zu-/Ablauf 11 für das Therapeutikum sowie die Zuleitungen 13 für den Reizstrom zugeordnet.

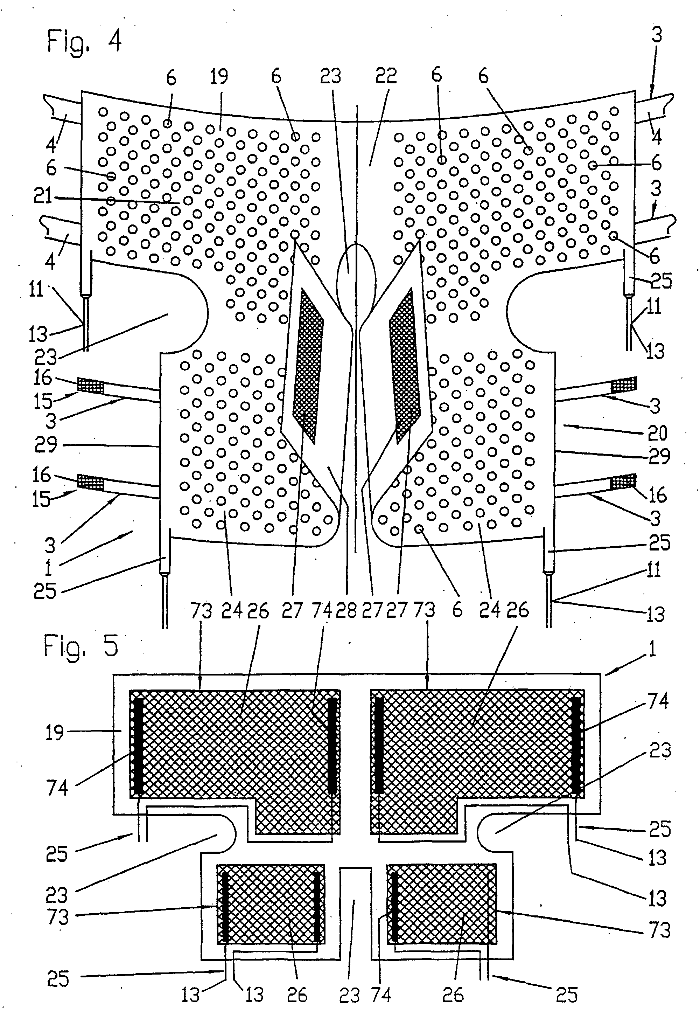

Die Therapiebandage 1 gemäß Fig. 4 weist im geschlossenen Zustand die Form einer Hose 19 auf. Mittels der Verschlüsse 3 wird die Hose 19 fixiert, wobei die Verschlüsse 3 am Hosenbein 20 Klettverschlüsse 15 und im Lendenbereich 21 Knopflochleisten 4 sind. Zur Bildung der Klettverschlüsse 15 sind an jedem Hosenbein 20 außenseitige Klettenbänder 16 vorgesehen, die mit einem auf einem Halterband 28 angeordneten Flauschband 27 zusammenwirken. Jedes Hosenbein 20 ist als Beinmanschette 24 ausgebildet, die sich direkt an den Lendenbereich 21 anschließt und auf der Therapiefläche 73 die Noppen 6 trägt. Die Therapiefläche 73 des Lendenbereiches 21 ist unter Freilassung eines Wirbelsäulenbereiches 22 ebenfalls mit ballige Noppen 6 versehen. Zwischen dem Lendenbereich 21 und den Hosenbeinen 20 sind Ausformungen 23 eingearbeitet, die eine Anpassung der Hose 19 an unterschiedliche Körperformen ermöglichen. Der Lendenbereich 21 weist ebenso wie die Hosebeine 20 jeweils zwei Zu-/Abläufe 11 sowie Reizstrom führende Zuleitungen 13 auf. Der Zu-/Ablauf 11 sowie die Zuleitung 13 sind zu einem gemeinsamen Anschluß 25 zusammengefaßt und stehen mit einer nicht dargestellten Versorgungseinrichtung für das Therapeutikum und den Reizstrom in Verbindung.

In der Darstellung der Hose 19 gemäß Fig. 5 ist die mit Noppens 6 versehene und durch eine Kreuzschraffur gekennzeichnete Therapiefläche 73 in mehrere Felder 26 aufgeteilt, wobei jedes Feld 26 mit zwei zueinander beabstandeten Elektroden 74 versehen ist, die mit Zuleitungen 13 zur Übertragung des Reizstromes in Verbindung stehen. Die Elektroden 74 können hierbei in einer Wandung 30 der Therapiefläche entweder in Alleinstellung oder zusätzlich zu den Noppen 6 vorgesehen werden.

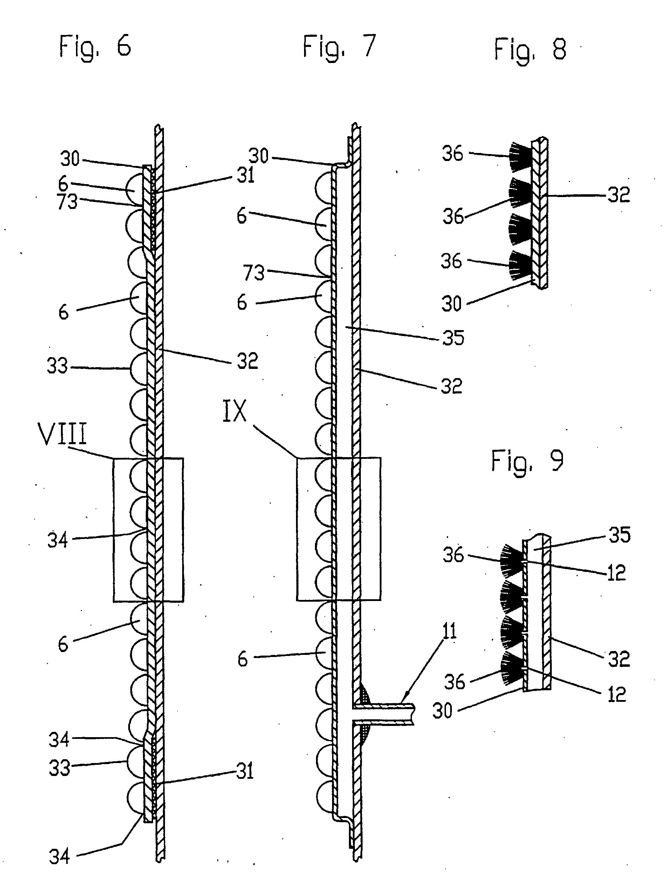

Gemäß dem in Fig. 6 dargestellten Schnitt durch die Therapiebandage 1 ist die die Noppen 6 tragende Wandung 30 der Therapiefläche 73 mittels Klettverbindungen 31 an der Außenwand 32 der Therapiebandage 1 festgelegt. Die Noppen 6 weisen auf der dem zu behandelnden Körperteil 76 zugewandten Seite eine steif ausgebildete Kuppe 33 auf. In einem Übergangsbereich 34 zwischen der. Kuppe 33 der Noppe 6 und der Therapiefläche 73 ist die Noppe 6 elastisch ausgeführt. Der elastische Übergangsbereich 34 bewirkt bei angelegter Therapiebandage 1 eine gewisse durch die Körperbewegung des Trägers der Therapiebandage 1 initierte Beweglichkeit der Kuppe 33 der Noppe 6 mit einer entsprechenden Massagewirkung und gewährleistet ein Rückstellen der Kuppe 33 bei nicht angelegter Therapiebandage 1.

Bei der Therapiebandage 1 nach Fig. 7 ist der Therapiefläche 73 unter Ausbildung eines Hohlraumes 35 die Außenwand 32 zugeordnet. Hierbei ist die Wandung 30 der Therapiefläche 73 umlaufend abgedichtet an der Außenwand 32 der Therapiebandage 1 festgelegt und die Außenwand 32 ist mit einem Zu-/Ablauf 11 für ein Therapeutikum versehen. Bei der Anordnung eines weiteren, nicht dargestellten Zu-/Ablaufes in der Außenwand 32 ist es möglich, das Therapeutikum im Kreislauf durch den Hohlraum 35 zuleiten.

Gemäß den Fig. 8 und 9 sind die Massageansätze 29 der Therapiebandage 1 als Bürsten 36 ausgebildet und an der Wandung 30 der Therapiefläche 73 befestigt. Im Zentrum der Bürsten 36 befindet sich jeweils eine Austrittsöffnung 12 in der Wandung 30, durch die ein über den Zu-/Ablauf 11 in den Hohlraum 35 eingeleitetes Therapeutikum auf die Haut der zu behandelnden Körperfläche geleitet wird. Die Benetzung der Haut erfolgt in Abhängigkeit von dem Durchmesser der Austrittsöffnung 12 und dem im Hohlraum 35 herrschenden Druck des Therapeutikums.

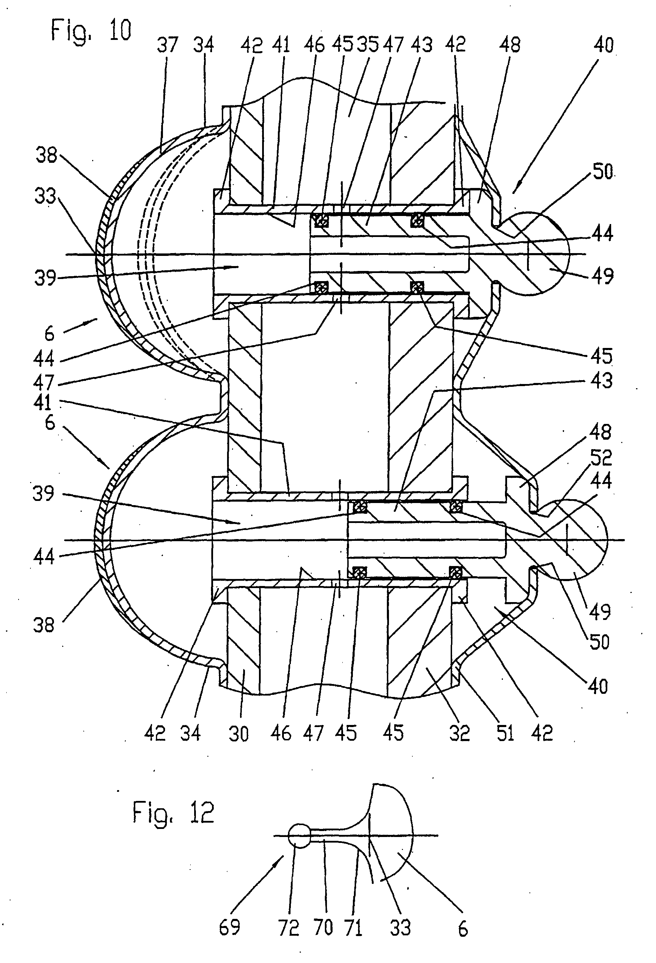

Die Darstellung nach Fig. 10 zeigt Noppen 6, die Bestandteile einer elastischen, an der Wandung 30 der Therapiefläche 73 abgedichtet befestigten Noppenfolie 37 sind. In Abhängigkeit von der Elastizität der Noppenfolie 37 und dem im Hohlraum 35 herrschenden Druck bilden sich, wie durch die gestrichelten Linien dargestellt, mehr oder weniger große bzw. steife Noppen 6 aus. Zwischen der mit einer Verdickung 38 versehenen Kuppe 33 der Noppe 6 und der Therapiefläche 73 ist der elastische Übergangsbereich 34 vorhanden.

Im Zentrum jeder Noppe 6 ist in die Wandung 30 der Therapiefläche 73 ein in den Hohlraum 35 mündender Kanal 39 eingelassen, der den Durchtritt des Therapeutikums aus den Hohlraum 35 in die Noppe 6 ermöglicht. In den Kanal 39 ist zum Öffnen bzw. Schließen desselben ein Ventil 40 eingesetzt, das ein Ventilrohr 41 mit jeweils endseitigem Ansatz 42 umfaßt, wobei die Ansätze 42 die Wandung 30, den Hohlraum 35 sowie die Außenwand 32 zwischen sich aufnehmen. In das Ventilrohr 41 ist ein Ventilstößel 43 eingesetzt, der zur Abdichtung gegen das Ventilrohr 41 zwei zueinander beabstandete, in entsprechenden Nuten 44 angeordnete Runddichtringe 45 trägt. Die Runddichtringe 45 gleiten entlang der Rohrwand 46 des Ventilrohrs 41 und schließen bzw. öffnen einen Durchlaß zwischen dem Hohlraum 35 und dem Ventilrohr 41 bzw. zwischen dem Hohlraum 35 und der zugeordneten Noppe 6, der über zwei in das Ventilrohr 41 eingebrachte Bohrungen 47 hergestellt ist.

Im geschlossenen Zustand des Ventils 40, in dem der Durchlaß zwischen dem Hohlraum 35 und der zugeordneten Noppe 6 versperrt ist, taucht der Ventilstößel 43 komplett in den Kanal 39 ein und ein Anschlag 48 des Ventilstößels 43 kommt an dem zugeordneten Ansatz 42 des Ventilrohrs 41 zur Anlage. Die auf dem Ventilstößel 43 angeordneten Runddichtringe 45 nehmen hierbei die in den Kanal 39 eingebrachten Bohrungen 47 zwischen sich auf. Am Anschlag 48 des Ventilstößels 43 ist unter Zwischenordnung einer Einschnürung 50 ein Knopf 49 angeformt. Auf der Außenwand 32 der Therapiebandage 1 ist eine elastische Matte 51 angeordnet, die mit einer der Anzahl vorhandener Ventile 40 entsprechenden Anzahl Löcher 52 versehen ist, wobei in jedem Loch 52 eine Einschnürung 50 eines zugeordneten Ventils 40 einliegt. Aufgrund der Vorspannung der elastischen Matte 51 befinden sich die Ventile 40 stets im geschlossenen Zustand, weshalb innerhalb des Noppens 6 ein konstanter Druck herrscht.

Zur Änderung des in dem Noppen 6 herrschenden Drucks wird der Ventilstößel 43 aus dem Ventilrohr 41 herausgezogen, bis der dem Noppen 6 zugewandte Runddichtring 45 die Bohrungen 47 freigibt und das Therapeutikum aus dem Hohlraum 35 in die Noppe 6 dringen kann. Das Öffnen des Ventils 40 erfolgt durch ein Angreifen an dem Knopf 49 gegen die von der Matte 51 auf den Anschlag 48 ausgeübte Rückstellkraft. Nach dem Loslassen des Knopfes 49 erfolgt ein selbsttätiges Rückstellen des Ventils 40 in den geschlossenen Zustand. Dem Ventil 40 kann auch eine nicht dargestellte Rasteinrichtung zugeordnet werden, die das Ventil 40 in der geöffneten Stellung hält. Somit wirkt sich eine Druckänderung innerhalb des Hohlraumes 35 direkt auf den zugeordneten Noppen 6 aus.

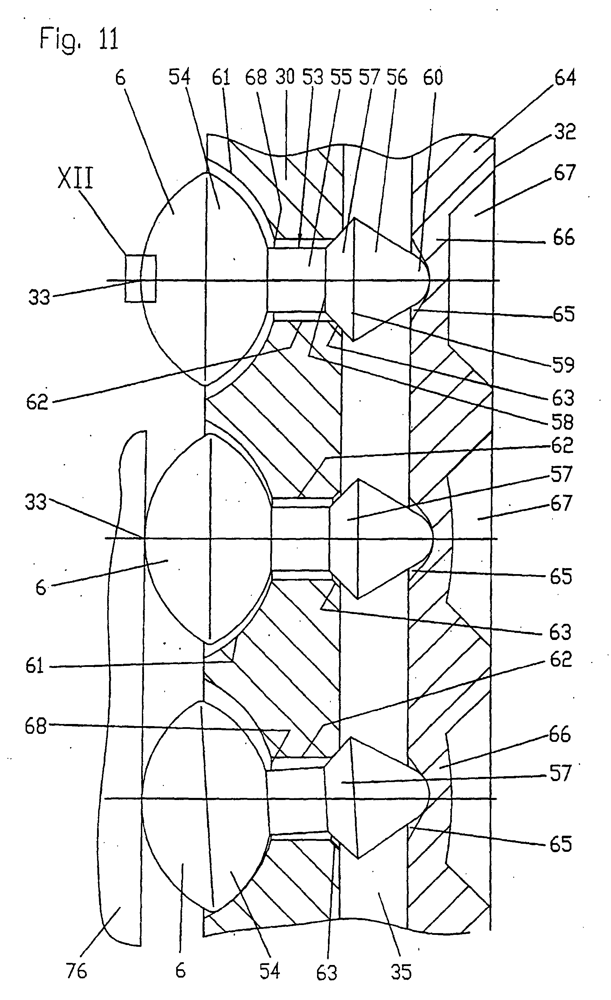

Die in Fig. 11 dargestellten Noppen 6 sind massiv ausgeführt und mittels eines Halters 53 in der Wandung 30 der Therapiefläche 73 gelagert. Der Halter 53 umfaßt ein an der Unterseite der Noppe 6 angeformtes Kugelsegment 54, einen sich koaxial daran anschließenden,. einen Dichtkegel 57 tragenden Halteransatz 55 sowie einen Druckkonus 56, der sich an den Dichtkegel 57 anschließt. Der Dichtkegel 57 ist mit seinem kleinsten Durchmesser 58, der dem des Halteransatzes 55 entspricht, an diesem angeformt und vergrößert seinen Durchmesser entgegen der Richtung der Noppe 6. Am größten Durchmesser 59 des Dichtkegels 57 schließt sich mit gleichem Durchmesser der Druckkonus 56 an, der die Form eines Kegelstumpfes mit einer verrundeten Spitze 60 aufweist. In die Wandung 30 der Therapiefläche 73 ist ein Noppenschale 61 eingelassen, in der das Kugelsegment 54 mit Spiel einliegt. An die Noppenschale 61 schließt sich eine koaxial, den Halteransatz 55 mit Spiel aufnehmende Ansatzbohrung 62 an. In entgegengesetzter Richtung zu der Noppenschale 61 weist die Wandung 30 einen Dichtsitz 63 mit einer Neigung, die der Neigung des Dichtkegels 57 des Noppenhalters 53 entspricht, auf. Auf der der Therapiefläche 73 entgegengesetzten Seite der Wandung 30 schließt sich der Hohlraum 35 an, der von einer parallel zu der Wandung 30 angeordneten Federmatte 64 begrenzt wird. Die im Querschnitt profilierte Federmatte 64 entspricht der Außenwand 32 der Therapiebandage 1 und beaufschlagt den Noppenhalter 53. Die Federmatte 64 weist im Bereich jedes Druckkonuses 56 eine Einbeulung 65 auf, wobei die Wandung 66 der Federmatte 64 im Bereich der Einbeulung 65 durch eine der Einbeulung 65 gegenüberliegende Vertiefung 67 zusätzlich verjüngt ist, um ein leichtes Einfedern der Noppe 6 bei Belastung zu ermöglichen. Zur Montage der Noppe 6, wird der Dichtkegel 57 des Halteransatzes 55 durch die sich elastisch erweiternde Ansatzbohrung 62 gedrückt. Nach dem Passieren des großen Durchmessers 59 des Dichtkegel 57 erfolgt eine Rückstellung der Ansatzbohrung 62, wonach die Noppe 6 unverlierbar in der Wandung 30 gehaltert ist.

Durch die Wirkung der Federmatte 64 wird der Dichtkonuns 56 in den Dichtkegel 57 gedrückt und die Ansatzbohrung 62 ist gegenüber dem Hohlraum 35 verschlossen. Erfolgt nun eine Belastung der Noppe 6 durch ein zu behandelndes Körperteil 76 in axialer Richtung, wird die Noppe 6 entgegen der Wirkung der Federmatte 64 teilweise in die Noppenschale 61 gedrückt. Dadurch entfernt sich der Dichtkegel 57 von dem Dichtkonus 56 und die Ansatzbohrung 62 ist geöffnet, weshalb ein Therapeutikum aus dem Hohlraum 35 dringen und die Haut des zu behandelnden Körperteiles 76 benetzen kann. Bei zunehmender Belastung der Noppe 6 kommt das Kugelsegment 54 auf dem umlaufenden Rand 68 der Ansatzbohrung 62 zu liegen und dichtet gegenüber der Ansatzbohrung 62 ab. Falls in dieser Stellung der Noppe 6 Therapeutikum aus dem Hohlraum 35 austreten soll, ist das Kugelsegment 54 mit axial verlaufenden Rillen zu versehenen. Erfährt die Noppe 6 eine nicht axial ausgerichtete Belastung durch das zu behandelndes Körperteil 76, kippt sie in. der Anlage zwischen dem Dichtkonus 56 und dem Dichtkegel 57 bis das Kugelsegment 54 teilweise in der Noppenschale 61 zur Anlage kommt. Hierbei wird die Ansatzbohrung 62 teilweise freigegebenen und das Therapeutikum kann aus dem Hohlraum 35 austreten. Wirkt keine Belastung auf die Noppen 6, erfolgt eine Rückstellung der Halteransätze 55 aufgrund der federnden Wirkung der Matte 51 und die Ansatzbohrungen 62 werden verschlossen.

Die Fig. 12 zeigt eine starke Vergrößerung der Kuppe 33 der Noppe 6, von.der zentrisch eine Akupunkturspitze 69 abgeht. Die Akupunkturspitze 69 umfaßt eine Kugel 72, die an einem Schaft 70 angeformt ist, wobei der Schaft 70 über einen Radius 71 mit der Kuppe 33 in Verbindung steht.

### Bezugszeichenliste

- 1: Therapiebandage
- 2: Weste
- 3: Verschluß
- 4: Knopflochleiste
- 5: Knopf
- 6: Noppe
- 7: Armdurchführung
- 8: Halsdurchtritt
- 9: Bundöffnung
- 10: Bund
- 11: Zu-/Ablauf
- 12: Austrittsöffnung
- 13: Zuleitung
- 14: Manschette
- 15: Klettverschluß
- 16: Klettenband
- 17: Reihe
- 18: Flächenbandage
- 19: Hose
- 20: Hosenbein
- 21: Lendenbereich
- 22: Wirbelsäulenbereich
- 23: Ausformung
- 24: Beinmanschette
- 25: Anschluß
- 26: Feld
- 27: Flauschband
- 28: Halterband
- 29: Massageansatz
- 30: Wandung
- 31: Klettverbindung
- 32: Außenwand
- 33: Kuppe
- 34: Übergangsbereich
- 35: Hohlraum
- 36: Bürste
- 37: Noppenfolie
- 38: Verdickung
- 39: Kanal
- 40: Ventil
- 41: Ventilrohr
- 42: Ansatz
- 43: Ventilstößel
- 44: Nut
- 45: Runddichtring
- 46: Rohrwand
- 47: Bohrung
- 48: Anschlag
- 49: Knopf
- 50: Einschnürung
- 51: Matte
- 52: Loch
- 53: Noppenhalter
- 54: Kugelsegment
- 55: Halteransatz
- 56: Druckkonus
- 57: Dichtkegel
- 58: Durchmesser
- 59: Durchmesser
- 60: Spitze
- 61: Noppenschale
- 62: Ansatzbohrung
- 63: Dichtsitz
- 64: Federmatte
- 65: Einbeulung
- 66: Wandung
- 67: Vertiefung
- 68: Rand
- 69: Akupunkturspitze
- 70: Schaft
- 71: Radius
- 72: Kugel
- 73: Therapiefläche
- 74: Elektrode
- 75: Gelenk
- 76: Körperteil

## Patentansprüche

1. Therapiebandage mit mindestens einer Therapiefläche (73) für ein zu behandelndes Körperteil (76), auf der in Richtung des zu behandelnden Körperteiles (76) weisende Massageansätze (29) angeordnet sind, wobei die Therapiebandage (1) mittels eines Verschlusses (3) an dem Körperteil (76) lösbar festgelegt ist, **dadurch gekennzeichnet, dass** die Massageansätze (29) als Bürsten (36) ausgebildet sind.

2. Therapiebandage mit mindestens einer Therapiefläche (73) für ein zu behandelndes Körperteil (76), auf der in Richtung des zu behandelnden Körperteiles (76) weisende, als ballige Noppen (6) ausgebildete Massageansätze (29) angeordnet sind, wobei die Therapiebandage (1) mittels eines Verschlusses (3) an dem Körperteil (76) lösbar festgelegt ist, **dadurch gekennzeichnet ,dass** auf der Kuppe (33) des Noppens (6) eine Akupunkturspitze (69) angeordnet ist.

3. Therapiebandage nach Anspruch 2, **dadurch gekennzeichnet, daß** jeder Noppen (6) mindestens im Bereich seiner Kuppe (33) steif ausgeführt ist.

4. Therapiebandage nach Anspruch 2 oder 3, **dadurch gekennzeichnet, daß** zwischen der Kuppe (33) des Noppens (6) und der Therapiefläche (73) ein elastischer Übergangsbereich (34) vorgesehen ist.

5. Therapiebandage nach einem der Ansprüche 1 bis 4, **dadurch gekennzeichnet, daß** der Therapiefläche (73) unter Ausbildung eines mit einem Zu-/Ablauf (11) für ein Therapeutikum versehenen Hohlraumes (35) eine Außenwand (32) zugeordnet ist.

6. Therapiebandage nach einem der Ansprüche 1 bis 5, **da-durch gekennzeichnet, daß** jedem Noppen (6) bzw. jeder Bürste (36) eine mit dem Hohlraum (35) verbundene Austrittsöffnung (12) für das Therapeutikum zugeordnet ist.

7. Therapiebandage nach einem der Ansprüche 1 bis 6, **dadurch gekennzeichnet, daß** jede Noppe (6) bzw. Bürste (36) in der Wandung (30) der Therapiefläche (73) gehaltert ist.

8. Therapiebandage nach Anspruch 7, **dadurch gekennzeichnet, daß** jede Noppe (6) bzw. Bürste (36) mit einem Halter (53) versehen ist, der ein an der Unterseite der Noppe (6) bzw. Bürste (36) angeformtes Kugelsegment (54), einen koaxial davon abgehenden, einen Dichtkegel (57) tragenden Halteransatz (55) sowie einen an den Dichtkegel (57) anschließenden Druckkonus (56) umfaßt, wobei in einem unbeaufschlagten Zustand der Massagenoppe (6) der Dichtkegel (57) aufgrund einer auf den Druckkonus (56) wirkenden Federmatte (64) an einem in Richtung der Außenwand (32) weisenden Dichtsitz (63) der Therapiefläche (73) anliegt, der Halteransatz (55) mit Spiel von einer Ansatzbohrung (62) aufgenommen ist und das Kugelsegment (54) beabstandet zu einer Noppenschale (61) in der Therapiefläche (73) einliegt.

9. Therapiebandage nach einem der Ansprüche 2 bis 7, **dadurch gekennzeichnet, daß** die Noppen (6) Bestandteil einer elastischen, an der Therapiefläche (73) befestigten Noppenfolie (37) sind, wobei jedem Noppen (6) ein in den Hohlraum (35) mündender Kanal (39) zugeordnet ist.

10. Therapiebandage nach Anspruch 9, **dadurch gekennzeichnet, daß** in den Kanal (39) ein Ventil (40) eingesetzt ist.

11. Therapiebandage nach Anspruch 10, **dadurch gekennzeichnet, daß** das Ventil (40) federbelastet oder mit einer Rasteinrichtung ausgeführt ist.

12. Therapiebandage nach einem der Ansprüche 2 bis 9, **dadurch gekennzeichnet, daß** im Bereich der Noppen (6) über Zuleitungen (13) mit Reizstrom beaufschlagbare Elektroden (74) vorgesehen sind.

13. Therapiebandage nach einem der Ansprüche 1 bis 12, **gekennzeichnet durch** die Ausbildung als Flächenbandage (18).

14. Therapiebandage nach einem der Ansprüche 1 bis 12, **gekennzeichnet durch** die Ausbildung als Weste (2), Hose (19) oder Manschette (14).

15. Therapiebandage nach Anspruch 1 oder 2, **dadurch gekennzeichnet, daß** der Verschluß (3) als mit mindestens einem Knopf (5) zusammenwirkende Knopflochleiste (4) oder als Klettverschluß (15) ausgebildet ist.

## Claims

1. Therapeutic bandage comprising at least one therapeutic surface (73) for a body part (76) to be treated, on which therapeutic surface (73) massaging projections (29) are disposed facing in the direction of the body part (76) to be treated, wherein the therapeutic bandage (1) is secured on the body part (76) in a removable manner by means of a closure (3), **characterised in that** the massaging projections (29) are in the form of brushes (36).

2. Therapeutic bandage comprising at least one therapeutic surface (73) for a body part (76) to be treated, on which therapeutic surface (73) massaging projections (29) in the form of spherical knobs (6) are disposed facing in the direction of the body part (76) to be treated, wherein the therapeutic bandage (1) is secured on the body part (76) in a removable manner by means of a closure (3), **characterised in that** an acupuncture point (69) is disposed on the peak (33) of the knob (6).

3. Therapeutic bandage according to claim 2, **characterised in that** each knob (6) is rigid at least in the region of its peak (33).

4. Therapeutic bandage according to claim 2 or 3, **characterised in that** a resilient transition region (34) is provided between the peak (33) of the knob (6) and the therapeutic surface (73).

5. Therapeutic bandage according to one of claims 1 to 4, **characterised in that** an outer wall (32) is associated with the therapeutic surface (73) with the forming of a cavity (35) provided with an inlet/outlet (11) for a therapeutic means.

6. Therapeutic bandage according to one of claims 1 to 5, **characterised in that** an outlet opening (12), which is connected to the cavity (35) for the therapeutic means, is associated with each knob (6) or respectively each brush (36).

7. Therapeutic bandage according to one of claims 1 to 6, **characterised in that** each knob (6) or respectively brush (36) is retained in the wall (30) of the therapeutic surface (73).

8. Therapeutic bandage according to claim 7, **characterised in that** each knob (6) or respectively brush (36) is provided with a holder (53), which holder (53) comprises a spherical segment (54) integrally formed on the underside of the knob (6) or respectively brush (36), a holder projection (55) which extends from the spherical segment in a coaxial manner and supports a sealing cone (57), as well as a pressure cone connected to the sealing cone (57), wherein, in a state where the massaging knob (6) is not being acted upon, the sealing cone (57), on account of a spring mat (64) working on the pressure cone (56), abuts against a sealing seat (63) of the therapeutic surface (73), which sealing seat (63) points in the direction of the outer wall (32), the holder projection (55) is accommodated with play by a projection bore (62) and the spherical segment (54) is secured at a spacing from a knob shell (61) in the therapeutic surface (73).

9. Therapeutic bandage according to one of claims 2 to 7, **characterised in that** the knobs (6) are component parts of a resilient knob film (37), which is secured to the therapeutic surface (73), wherein a channel (39), which terminates in the cavity (35), is associated with each knob (6).

10. Therapeutic bandage according to claim 9, **characterised in that** a valve (40) is inserted in the channel (39).

11. Therapeutic bandage according to claim 10, **characterised in that** the valve (40) is spring-loaded or is designed with a locking mechanism.

12. Therapeutic bandage according to one of claims 2 to 9, **characterised in that** electrodes (74), which can be acted upon by stimuli via supply lines (13), are provided in the region of the knobs (6).

13. Therapeutic bandage according to one of claims 1 to 12, **characterised by** the form of a surface bandage (18).

14. Therapeutic bandage according to one of claims 1 to 12, **characterised by** the form of a jacket (2), trousers (19) or sleeve (14).

15. Therapeutic bandage according to claim 1 or 2, **characterised in that** the closure (3) is in the form of buttonhole tape (4) interacting with at least one button (5) or in the form of Velcro ® fastener (15).

## Revendications

1. Bandage thérapeutique avec au moins une surface thérapeutique (73) pour une partie corporelle (76) à traiter, sur laquelle sont disposés des appendices de massage (29) dirigés vers la partie corporelle (76) à traiter, le bandage thérapeutique (1) étant fixé de manière amovible sur la partie corporelle (76) au moyen d'une fermeture (3), **caractérisé en ce que** les appendices de massage (29) sont réalisés sous la forme de brosses (36).

2. Bandage thérapeutique avec au moins une surface thérapeutique (73) pour une partie corporelle (76) à traiter, sur laquelle sont disposés des appendices de massage (29) réalisés sous la forme de boutons bombés (6) dirigés vers la partie corporelle (76) à traiter, le bandage thérapeutique (1) étant fixé de manière amovible sur la partie corporelle (76) au moyen d'une fermeture (3), **caractérisé en ce qu'**au sommet (33) du bouton (6) est disposée une aiguille d'acupuncture (69).

3. Bandage thérapeutique selon la revendication 2, **caractérisé en ce que** chaque bouton (6) est réalisé rigide au moins dans la zone de son sommet (33).

4. Bandage thérapeutique selon la revendication 2 ou 3, **caractérisé en ce qu'**une zone de transition élastique (34) est prévue entre le sommet (33) du bouton (6) et la surface thérapeutique (73).

5. Bandage thérapeutique selon l'une des revendications 1 à 4, **caractérisé en ce qu'**à la surface thérapeutique (73) est associée une paroi extérieure (32) formant une cavité (35) pourvue d'une arrivée/sortie (11) pour une substance thérapeutique.

6. Bandage thérapeutique selon l'une des revendications 1 à 5, **caractérisé en ce qu'**à chaque bouton (6) ou chaque brosse (36) est associé un orifice de sortie (12), relié à la cavité (35), pour la substance thérapeutique.

7. Bandage thérapeutique selon l'une des revendications 1 à 6, **caractérisé en ce que** chaque bouton (6) ou brosse (36) est maintenu dans la paroi (30) de la surface thérapeutique (73) .

8. Bandage thérapeutique selon la revendication 7, **caractérisé en ce que** chaque bouton (6) ou brosse (36) est pourvu d'un support. (53) qui comprend un segment sphérique (54) formé sur la face inférieure du bouton (6) ou de la brosse (36), un appendice de support (55) portant un cône d'étanchéité (57) partant coaxialement dudit segment sphérique, ainsi qu'un cône de pression (56) se raccordant au cône d'étanchéité (57), dans un état non sollicité du bouton de massage (6) le cône d'étanchéité (57) s'appliquant, en raison d'un mat élastique (64) agissant sur le cône de pression (56), contre un siège d'étanchéité (63) dirigé vers la paroi extérieure (32) de la surface thérapeutique (73), l'appendice de support (55) étant reçu avec jeu par un trou pour appendice (62) et le segment sphérique (54) se trouvant à l'intérieur de la surface thérapeutique (73) et distant d'une coupelle de bouton (61).

9. Bandage thérapeutique selon l'une des revendications 2 à 7, **caractérisé en ce que** les boutons (6) font partie intégrante d'une feuille à boutons (37) élastique, fixée sur la surface thérapeutique (73), à chaque bouton (6) étant associé un canal (39) débouchant dans la cavité (35).

10. Bandage thérapeutique selon la revendication 9, **caractérisé en ce qu'**une soupape (40) est insérée dans le canal (39).

11. Bandage thérapeutique selon la revendication 10, **caractérisé en ce que** la soupape (40) est soumise à l'action d'un ressort ou est réalisée avec un dispositif d'encliquetage.

12. Bandage thérapeutique selon l'une des revendications 2 à 9, **caractérisé en ce que** dans la zone des boutons (6) sont prévues des électrodes (74) qui peuvent être soumises à un courant de stimulation par des lignes d'arrivée (13) .

13. Bandage thérapeutique selon l'une des revendications 1 à 12, **caractérisé par le fait qu'**il est réalisé en tant que bandage plat (18).

14. Bandage thérapeutique selon l'une des revendications 1 à 12, **caractérisé en ce qu'**il est réalisé sous la forme d'un gilet (2), d'une culotte (19) ou d'un manchon (14).

15. Bandage thérapeutique selon la revendication 1 ou 2, **caractérisé en ce que** la fermeture (3) est réalisée sous la forme d'une bande à boutonnière (4) coopérant avec un bouton (5) ou sous la forme d'une fermeture à crochet (15).
